# EUROPÄISCHE PATENTANMELDUNG

(11) **EP 0 657 459 A1**
(43) Veröffentlichungstag der Anmeldung: **14.06.1995**
(21) Anmeldenummer: 94118276.8
(22) Anmeldetag: 21.11.1994
(51) Int. Cl.: C07D 513/04, A61K 31/54

(54) **Neue Thienothiazinderivate, ein Verfahren zu ihrer Herstellung und ihre Verwendung als Antiinflammatorikum und Analgeticum**

(30) Priorität: 06.12.1993 AT 2461/93
(71) Anmelder: Chemisch Pharmazeutische Forschungsgesellschaft m.b.H., A-4020 Linz (AT)
(72) Erfinder: Binder, Dieter Prof. Dr., A-1190 Wien (AT); Weinberger, Josef Dipl. Ing. Dr., A-1020 Wien (AT)

(57) **Zusammenfassung**

Thienothiazinderivate der Formel I 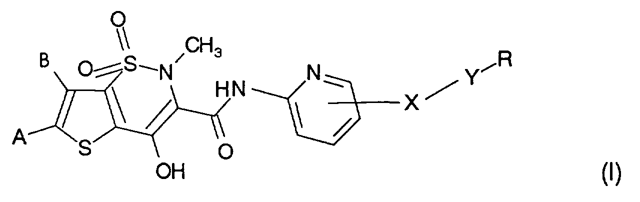
in der X eine Einfachbindung oder einen 5 - 12 gliedrigen, mono- oder polycyclischen, gegebenenfalls teilweise hydrierten Aryl- oder Heteroarylrest, der gegebenfalls durch Halogen, Niederalkyl oder Niederalkoxy substituiert sein kann,
Y eine Einfachbindung oder ein Heteroatom
R einen mono- oder polycyclischen, gegebenenfalls teilweise hydrierten 5 - 12 gliedrigen Aryl- oder Heteroarylrest bedeutet, der gegebenenfalls ein- oder mehrfach durch Halogen, Niederalkyl oder Niederalkoxy substituiert sein kann, A Halogen
und
B Wasserstoff oder Halogen bedeutet, ein Verfahren zu deren Herstellung und deren Verwendung als Pharmazeutika.

## Beschreibung

Die Erfindung betrifft neue Thienothiazinderivate, ein Verfahren zu ihrer Herstellung und ihre Verwendung.

Aus US 4,180,662 sind Thienothiazinderivate bekannt, die am Pyridinkern unsubstituiert sind. Diese Verbindungen weisen eine gute Cyclooxygenasehemmung auf.
Ferner sind aus Chemical Abstracts, Band 11, Nr.19, 166771 n und Chemical Abstracts, Band 115, Nr. 3, 21532 h Thienothiazinderivate bekannt, die am Pyridinkern unsubstituiert sind oder durch ein Hydroxylgruppe substituiert sind. Auch diese Verbindungen weisen eine gute Cyclooxygenasehemmung auf.

Es konnten nun neue am Pyridinkern substituierte Thienothiazinderivate gefunden werden, die bei weitgehender Beibehaltung der Cyclooxygenasehemmung eine signifikant erhöhte Hemmung der 5-Lipoxygenase bewirken.

Gegenstand der Erfindung sind demnach Thienothiazinderivate der Formel I 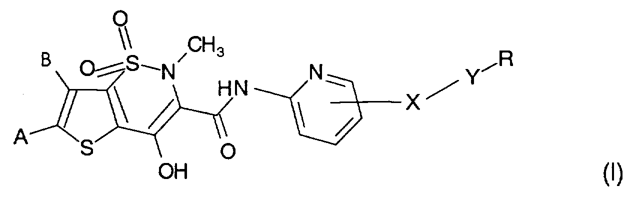
in der X eine Einfachbindung oder einen 5 - 12 gliedrigen, mono- oder polycyclischen, gegebenenfalls teilweise hydrierten Aryl- oder Heteroarylrest, der gegebenfalls durch Halogen, Niederalkyl oder Niederalkoxy substituiert sein kann,
Y eine Einfachbindung oder ein Heteroatom
R einen mono- oder polycyclischen, gegebenenfalls teilweise hydrierten 5 - 12 gliedrigen Aryl- oder Heteroarylrest bedeutet, der gegebenenfalls ein- oder mehrfach durch Halogen, Niederalkyl oder Niederalkoxy substituiert sein kann, A Halogen
und
B Wasserstoff oder Halogen bedeutet.

Bevorzugt sind jene Verbindungen, bei denen der Substituent an der 6 - Stellung des Pyridins verknüpft ist.

In der Formel (I) bedeutet X eine Einfachbindung oder einen 5 - 12 gliedrigen, mono- oder polycyclischen, gegebenenfalls teilweise hydrierten Aryl- oder Heteroarylrest. Solche Reste wären beispielsweise 5 - 12 gliedrige, mono- oder polycyclische, gegebenenfalls teilweise hydrierte aromatische oder heteroaromatische Reste, beispielsweise ein Phenyl- ein Thienyl-, ein Furyl-, ein Pyrrolyl-, ein Pyrimidyl-, ein Pyranyl-, ein Thiadiazinyl-, ein Azepinylrest, ein Chinolinylrest und dergleichen.
Diese Reste können gegebenenfalls ein- oder mehrfach durch Halogen, beispielsweise F, Cl, oder J oder durch Niederalkyl oder Niederalkoxy substituiert sein.
Niederalkyl bedeutet einen geradkettigen oder verzweigten Alkylrest mit 1 - 4 C- Atomen, beispielsweise einen Methyl-, einen Ethyl-, einen Propyl-, einen i-Propylrest, einen Butylrest, einen i-Butylrest oder einen t-Butylrest.
Niederalkoxy bedeutet einen geradkettigen oder verzweigten Alkoxyrest mit 1 - 4 C -Atomen, beispielsweise Methoxy, Ethoxy, Propoxy, i-Propoxy-, n-Butoxy-, i-Butoxy oder t-Butoxy.
Bevorzugt sind solche Verbindungen, bei denen X eine Einfachbindung oder einen 5 - 7 gliedrigen Aryl- oder Heteroarylrest, beispielsweise einen Phenylrest oder einen Furylrest bedeutet
Y bedeutet in der Formel (I) eine Einfachbindung oder ein Heteroatom, beispielsweise O, N oder S.
Bevorzugt sind jene Verbindungen, in denen Y eine Einfachbindung oder O bedeutet.

R bedeutet einen mono- oder polycyclischen, gegebenenfalls teilweise hydrierten, 5-12 gliedrigen Aryl- oder Heteroarylrest. Dies sind 5 - 12 gliedrige, gegebenenfalls teilweise hydrierte aromatische oder heteroaromatische Reste, beispielsweise ein Phenyl- ein Thienyl-, ein Furyl-, ein Pyrrolyl-, ein Pyrimidyl-, ein Pyranyl-, ein Thiadiazinyl-, ein Azepinylrest oder ein Benzofuryl-, ein Chinolinyl- oder ein Benzothienylrest.
Diese Reste können gegebenenfalls ein- oder mehrfach durch Halogen, beispielsweise F, Cl, Br oder durch Niederalkyl oder Niederalkoxy substituiert sein.

Bevorzugt sind jene Verbindungen, in denen R einen gegebenenfalls ein- oder mehrfach halogenierten Phenyl-, einen Benzofuryl oder einen Chinolinylrest bedeutet.

A kann Halogen, beispielsweise Cl, F, Br sein.
B bedeutet Wasserstoff oder Halogen, beispielsweise Cl, F oder Br.

Ein weiterer Gegenstand der Erfindung ist ein Verfahren zur Herstellung von Verbindungen der Formel (I), das dadurch gekennzeichnet ist, daß man eine Verbindung der Formel (II) 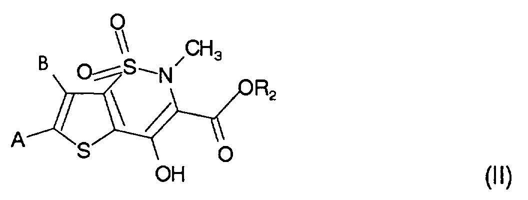
in der R₂ eine geradkettige oder verzweigte Alkylgruppe mit 1 - 4 C-Atomen bedeutet und A und B die oben genannte Bedeutung haben, mit einer Verbindung der Formel (III) 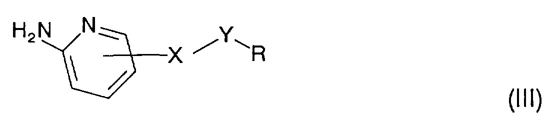
in der X, Y, und R die oben genannte Bedeutung haben, umsetzt und die so erhaltenen Verbindungen der Formel (I) gegebenenfalls in ihre pharmazeutisch verwendbaren Salze überführt.

Die Verbindungen der Formeln II und III sind literaturbekannt oder können analog dazu nach üblichen und dem Fachmann geläufigen Methoden hergestellt werden.

Das erfindungsgemäße Verfahren kann vorzugsweise auf eine der folgenden Weisen durchgeführt werden, indem man
a) eine Verbindung der Formel II in einem inerten Lösungsmittel, wie Diethylether, Dioxan, Toluol, Benzol oder dergleichen löst und bei einer Temperatur von - 20°C bis 100°C ein Äquivalent einer starken Base, wie Butyllithium oder LDA, in einem inerten Lösungsmittel, beispielweise n-Hexan, gegebenenfalls unter Inertgas zusetzt, zu dieser Salzlösung 1-10 Äquivalente einer Verbindung der Formel III zusetzt, mindestens 1 Äquivalent der starken Base zusetzt und zwischen 0,5 und 60 Stunden, vorzugsweise 1 - 48 Stunden bei - 20°C bis 100°C, rührt,
   oder
b) die Verbindungen der Formel II und der Formel III in einem inerten hochsiedenden Lösungsmittel, wie Toluol, Xylol, Pyridin, Chinolin, Dimethylformamid, Dimethylsulfoxid oder Hexamethylphosphorsäuretriamid löst und dieses Gemisch 1 - 30 Stunden auf 100°C bis 200°C erhitzt.
Die so erhaltenene Verbindungen der Formel I können gegebenenfalls leicht beispielsweise durch umkristallisieren, gereinigt werden.

Die Verbindungen der allgemeinen Formel I sind saure Verbindungen und können auf übliche Weise mit anorganischen oder organischen Basen in ihre pharmazeutische verwendbaren Salze übergeführt werden.
Die Salzbildung kann beispielsweise durchgeführt werden, indem man die Verbindungen der Formel I in einem geeigneten Lösungsmittel, z.B. Wasser, einem niederen aliphatischen Alkohol, beispielsweise Methanol, Ethanol und dergleichen, in Tetrahydrofuran, Dioxan, Benzol, Diethylether, Dimethylformamid oder Dimethylsulfoxid löst, eine äquivalente Menge der gewünschten Base zusetzt und nach beendeter Salzbildung das Lösungsmittel unter Vakuum abdestilliert.
Gegebenenfalls können die Salze nach ihrer Isolierung weiter gereinigt werden, beispielsweise durch umkristallisieren.

Als pharmazeutisch verträgliche Salze kommen beispielsweise Metallsalze, insbesondere Alkalimetallsalze und Erdalkalimetallsalze, wie Natrium, Magnesium, Kalium oder Calciumsalze in Frage. Weitere pharmazeutisch verwendbare Salze sind leicht kristallisierbare Ammoniumsalz, die sich von Ammoniak oder organischen Aminen, wie beispielsweise Mono-, Di- oder Tri- (Niederalkyl, Cycloalkyl-, oder Hydroxyalkyl-)Aminen, Niederalkylendiaminen, (Hydroxy-niederalkyl)- oder (Aryl-niederalkyl)- niederalkylammoniumbasen ab, beispielsweise Methylamin, Diethylamin, Triethylamin, Dicyclohexylamin, Triethanolamin, Ethylendiamin, Tris(hydroxy-methyl)-aminomethan, Benzyltrimethylammoniumhydroxid und dergleichen.

Die erfindungsgemäßen Verbindungen der Formel I und ihre Salze sind oral wirksam und zeigen im Vergleich zu am Pyridinkern unsubstituierten Verbindungen, wie beispielsweise dem aus US 4,180,662 bekannten 6-Chlor-4-hy-droxy-2-methyl-N-(2-pyridyl)-2H-thieno[2,3-e]1,2-thiazin-3-carboxamid-1,1-dioxid ("Lornoxicam" ) überraschenderweise eine signifikant höhere Hemmung der 5-Lipoxygenase, wobei die Cyclooxygenasehemmung weitgehend beibehalten wird.

Sie sind daher zur Behandlung von Beschwerden, die durch das natürliche Produkt der 5- Lipoxygenase, nämlich dem Leukotrien-B₄, mitverursacht werden, wie z.B. Entzündungen und Schmerz bei allergischem Asthma, Arthritis, Hautallergie usw. besonders gut geeignet.

Aufgrund dieser pharmakologischen Eigenschaften können die neuen Verbindungen allein oder in Mischung mit anderen Wirkstoffen in Form üblicher galenischer Zubereitungen als Heilmittel zur Behandlung von Erkrankungen, die durch Hemmung der 5-Lipoxygenase geheilt oder gelindert werden können, Verwendung finden.

Die Erfindung betrifft ferner Heilmittel, die beispielsweise in Form pharmazeutischer Zubereitungen Verwendung finden, welche die erfindungsgemäßen Verbindungen der Formel (I) oder ihre pharmezeutisch verwendbaren Salze in Mischung mit einem für die orale, enterale, parenterale oder topicale Applikation geeigneten pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline und dergleichen enthalten.

Die pharmazeutischen Präparate können in fester Form, beispielsweise als Tabletten, Filmtabletten, Dragees, Suppositoren, Kapseln, Mikrokapseln, oder in flüssiger Form, beispielsweise als Lösungen, Injektionslösungen, Suspensionen oder Emulsionen oder in Zusammensetzungen mit verzögerter Freigabe des Wirkstoffes vorliegen.
Gegebenenfalls können sie sterilisiert werden und/oder sie enthalten Hilfsstoffe, wie Konservierungs-, Stabilisierungs- oder Emulgiermittel, Salze zur Veränderung des osmotischen Drucks oder Puffer.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen in Kombination mit den oben genannten Hilfs- und/oder Trägerstoffen zu Kombinationspräparaten formuliert werden.

Die neuen Verbindungen können in den erfindungsgemäßen Zusammensetzungen in einem Anteil von 4 - 200 mg pro Tablette vorhanden sein, wobei der Rest ein pharmazeutisch verträglicher Füllstoff ist.

Eine geeignete Dosis zur Verabreichung der Verbindungen beträgt etwa 4 - 200 mg/kg pro Tag, jedoch kommen je nach Zustand des zu behandelenden Patienten auch andere Dosen in Frage. Die neuen Verbindungen können in mehreren Dosen aund auf oralem Weg verabreicht werden.

### Beispiel 1

### N-[6-(2-Benzo[b]thienyl)-2-pyridinyl]-6-chlor-4-hydrory-2-methyl-2H-thieno[2,3-e][1,2]thiazin-3-carboxamid-1,1-dioxid

900 mg (3,98 mmol) 6-(2-Benzo[b]thienyl)-2-pyridinamin und 1236 mg (3,99 mmol) 6-Chlor-4-hydroxy-2-methyl-2H-thieno[2,3-e][1,2]thiazin-3-carbonsäure-methylester-1,1-dioxid werden in 25 ml abs.Xylol 10 Stunden zum Sieden erhitzt. Die Lösung wird abgekühlt und der Niederschlag filtriert und mehrmals mit Aceton digeriert. Dieses Rohprodukt wird aus Toluol/Aktivkohle umkristallisiert.
Ausbeute: 1,37 g gelbe Kristalle (68% der Theorie)
Schmelzpunkt: 257 - 261 °C (Zers., Toluol)
Dünnschichtchromatogramm: CH₂Cl₂:MeOH 40:1 R_{F} = 0,70
¹H-NMR: (Trifluoressigsäure-d1)
δ(ppm): 8,03 (m, 1H, Py-H4); 7,69 (s, 1H, Tz-H5); 7,40-7,65 (m, 3H, Th-H4 + Th-H7 + Py-H5); 7,00-7,25 (m, 3H, Th-H5 + Th-H6 + PY-H3); 6,95 (s, 1H, Th-H3); 2,70 (s, 3H, CH3)
¹³C-NMR: (Trifluoressigsäure-d1)
δ(ppm): 173,58 (s, CO); 162,38 (s, Py-C2); 151,55 (s, Tz-C4); 146,73 (s, Th-C7a*); 146,26 (s, Tz-C4a); 144,95 (s, Th-C2*); 143,02 (d, Py-C4); 142,13 (s, Tz-C7a); 135,26 (s, Tz-C6); 134,63 (s, Th-C3a); 131,89 (d, Th-C4); 130,90 (s, Py-C6); 129,80 (d, Th-C5*); 129,33 (d, Th-C6*); 126,52 (d, Tz-C7); 126,04 (d, Th-C7*); 122,91 (s, Tz-C3); 122,35 (d, Py-C3); 117,56 (d, Py-C5); 43,21 (q, CH3)
Das Ausgangsmaterial kann wie folgt hergestellt werden

### 2-Benzo[b]thienyl-tributylstannan

8,498 g (63,3 mmol) Benzo[b]thiophen in 100 ml abs. THF werden bei -20 °C mit 31 ml (77,5 mmol) n-Butyllithium versetzt, eine Stunde gerührt, auf -70 °C gekühlt und 20,756 g (63,8 mmol) Tributylzinnchlorid in 20 ml abs. THF zugegeben.
Die Lösung läßt man auf Raumtemperatur erwärmen und zieht das Lösungsmittel ab. Der Rückstand wird zwischen 400 ml Wasser und 100 ml Ether verteilt, die wäßrige Phase mit 5x100ml Ether extrahiert, die vereinten organischen Phasen mit Wasser gewaschen, getrocknet und das Lösungsmittel abgezogen.
Das erhaltene Rohprodukt wird einer Flashchromatographie (300 g KG60, PE)unterworfen. Ausbeute: 22,55 g farblose Flüssigkeit (84% der Theorie)
Dünnschichtchromatogramm:
PE:EE 1:1 R_{F} = 0,90
n-Hx R_{F} = 0,45
PE R_{F} = 0,50
¹H-NMR: (CDCl₃)
δ(ppm): 7,90 (d, 1H, Th-H4); 7,85 (d, 1H, Th-H7); 7,40 (s, 1H, Th-H3); 7,30 (m, 1H, Th-H5); 7,25 (m, 1H, Th-H6); 1,65 (m, 6H, β-CH3); 1,40 (m, 6H, χ-CH2); 1,20 (t, 6H, δ-CH2); 0,95 (t, 9H, α-CH3)
¹³C-NMR: (CDCl₃)
δ(ppm): 144,32 (s, Th-C2*); 141,04 (s, Th-C7a*); 139,70 (s, Th-C3a*); 132,03 (d, Th-C3); 123,64 (d, Th-C5*); 123,26 (d, Th-C4*); 122,68 (d, Th-C6*); 121,78 (d, Th-C7*); 29,05 (t, χ-C*); 27,33 (t, β-C*): 13,70 (q, α-C); 10,84 (t, δ-C*)

### 2-(2-Benzo[b]thienyl)-6-brompyridin

20,02 g (47,3 mmol) 2-benzo[b]thiophen-tributylstannan, 11,23 g (47,4 mmol) 2,6-Dibrompyridin, 4,79 g (47,3 mmol) Triethylamin, 0,496 g (1,89 mmol) Triphenylphosphin und 0,213 g (0,95 mmol) Palladiumdiazetat werden in 300 ml abs DMF eine Stunde bei 105°C gerührt. Das Reaktionsgemisch wird auf 600 ml Salzsäure (1,6N) gegossen und der Niederschlag abfiltriert. Das Rohprodukt wird aus Toluol/Aktivkohle umkristallisiert und ohne weitere Trennung in die nächste Stufe eingesetzt.
Ausbeute: 4,94 g farblose Kristalle (36% der Theorie)
Schmelzpunkt: 163 - 168°C (Toluol)
Dünnschichtchromatogramm: PE:EE 40:3 R_{F} = 0,35
¹H-NMR: (DMSO-d6)
δ(ppm): 8,20 (s, 1H, Th-H3); 8,10 (d, 1H, Py-H3); 8,00 (m, 1H, Th-H4); 7,90 (m, 1H, Th-H7); 7,85 (dd, 1H, Py-H4); 7,60 (d, 1H, Py-H5); 7,40 (m, 1H, Th-H5); 7,40 (m, 1H, Th-H6)
¹³C-NMR: (DMSO-d6)
δ(ppm): 152,87 (s, Th-C7a); 142,27 (s, Py-C6); 140,87 (s, Th-C3a); 140,16 (d, Py-C4); 139,96 (s, Py-C2); 126,96 (d, Py-C3); 125,57 (d, Th-C4); 124,78 (d, Th-C5); 124,45 (d, Th-C6); 123,05 (d, Th-C7); 122,60 (d, Py-C5); 118,90 (d, Th-C3)

### 6-(2-Benzo[b]thienyl)-2-pyridinamin

3,47 g (12,0 mmol) 2-(2-Benzo[b]thienyl)-6-brompyridin und 2,63 g (67,4 mmol) Natriumamid werden in 200 ml Ammoniak eine Stunde bei 0°C in einem Autoklaven gerührt.
Nach Abkühlen des Autoklaven auf -50 °C quenscht man mit 3,73 g (69,7 mmol) Ammoniumchlorid und verdampft langsam den Ammoniak. Der Rückstand wird zwischen Wasser und EE verteilt, 8x mit insgesamt 1100 ml EE extrahiert, die organische Phase 2x mit Wasser gewaschen, getrocknet, bis zur Kristallisation eingeengt, abgekühlt und die Kristalle des Nebenproduktes aus der vorigen Stufe abfiltriert.
Die Mutterlauge wird vollständig eingedampft, der Rückstand wird 2x mit jeweils 5 ml HCl-gesättigtem Methanol digeriert und eingedampft, 2x mit reinem Methanol digeriert und eingedampft, das Hydrochlorid mit 20 ml kaltem Benzol digeriert und abfiltriert und das Amin durch Verteilen zwischen EE und gesättigter Natriumhydrogencarbonatlösung wieder in Freiheit gesetzt.
Ausbeute: 1,30 g hellbraune Kristalle (48% der Theorie)
Schmelzpunkt: 225-231 °C (Hydrochlorid)
Dünnschichtchromatogramm:
PE:EE 40:3 R_{F} = 0,15
PE:EE 1:1 R_{F}=0,70
¹H-NMR: als Hydrochlorid
δ(ppm): 8,50 (s, 1H, Th-H3); 8,05 (m, 1H, Py-H4*); 7,90 (m, 2H, Th-H4* + Th-H7*); 7,45 (m, 2H, Th-H5* + Th-H6*); 7,10 (d, 1H, Py-H3); 6,95 (d, 1H, Py-H5)

### Beispiel 2

### N-[6-(4-Biphenyl)-2-pyridinyl]-6-chlor-4-hydroxy-2-methyl-2H-thieno[2,3-e][1,2]thiazin-3-carboxamid-1,1-dioxid

609 mg (2,47 mmol) 6-(4-Biphenyl)-2-pyridinamin und 767 mg (2,48 mmol) 6-Chlor-4-hydroxy-2-methyl-2H-thieno[2,3-e][1,2]thiazin-3-carbonsäuremethylester-1,1-dioxid werden in 15 ml Xylol 6 Stunden zum Sieden erhitzt. Das Rohprodukt nach Erkalten abfiltriert und aus DMF/Aktivkohle umkristallisiert.
Ausbeute: 1,07 g hellgelbe Kristalle (83% der Theorie)
Schmelzpunkt: 242-245 °C (Zers., DMF)
Dünnschichtchromatogramm:
CH₂Cl₂:MeOH 40:1 R_{F} = 0,50
CH₂Cl₂:MeOH 40:3 R_{F} = 0,90
¹H-NMR: (Trifluoressigsäure-d1)
δ(ppm): 8,05 (dd, 1H, Py-H4); 7,60 (m, 4H, Bz-H2 + Bz-H3); 7,50 (s, 1H, Tz-H7); 7,25 (m, 3H, Py-H3* + Ph-H2); 7,05 (m, 3H, Ph-H4 + Ph-H3); 7,00 (d, 1H, Py-H5); 2,75 (s, 3H, CH3)
¹³C-NMR: (Trifluoressigsäure-d1)
δ(ppm): 173,56 (s, CO); 162,44 (s, Py-C2); 152,61 (s, Py-C6); 151,73 (d, Py-C4); 150,28 (s, Tz-C4); 146,51 (s, Tz-C4a); 142,45 (s, Bz-C1); 141,81 (s, Tz-C7a); 135,46 (s, Tz-C6); 132,65 (d, Bz-C3 + Bz-C5); 132,34 (d, Bz-C2 + Bz-C6); 131,54 (d, Bz-C4); 130,53 (d, Ph-C3 + Ph-C5); 130,44 (d, Ph-C2 + Ph-C6); 126,48 (d, Tz-C7); 122,77 (d, Py-C3); 117,44 (d, Py-C5); 112,66 (s, Tz-C3); 43,21 (q, CH3)
Das Ausgangsmaterial kann wie folgt hergestellt werden

### 4-Biphenylboronsäure

18,034 g (77,4 mmol) 4-Brombiphenyl in 200 ml THF werden bei -70 °C mit 35,0 ml (87,5 mmol) n-Butyllithium versetzt. Nach Abkühlen der Lösung auf - 100 °C tropft man 16,50 g (159 mmol) Borsäuretrimethylester (verdünnt mit 10 ml THF) zu und läßt auf Raumtemperatur erwärmen.
Die Reaktionslösung wird mit 500 ml 1N Salzsäure versetzt, mit 3x120 ml Diethylether extrahiert, die organische Phase 2x mit Wasser gewaschen, getrocknet, filtriert und das Lösungsmittel abgezogen. Das Rohprodukt wird mit DiPE kristallisiert und abfiltriert.
Ausbeute: 11,30 g farblose Kristalle (74% der Theorie)
Schmelzpunkt: 216-220 °C (DIPE)
Dünnschichtchromatogramm: PE:EE 1:1 R_{F} = 0,50
¹H-NMR: (Aceton-d6)
δ(ppm): 7,95 (d, 2H, Bz-H2); 7,70 (d, 2H, Ph-H2*); 7,65 (d, 2H, Bz-H3*); 7,40 (d, 1H, Ph-H4*); 7,35 (dd, 2H, Ph-H3*)
¹³C-NMR: (Aceton-d6)
δ(ppm): 141,16 (s, Ph-C1*); 140,44 (s, Bz-C4*); 134,76 (d, Bz-C2* + Bz-C6*); 134,11 (d, Bz-C3* + Bz-C5*); 128,88 (d, Ph-C3* + Ph-C5*); 126,67 (d, Ph-C2* + Ph-C6*); 125,80 (d, Ph-C4*)

### 2-(4-Biphenyl)-6-brom-pyridin

7,99 g (40,3 mmol) 4-Biphenylboronsäure, 9,67 g (40,8 mmol) 2,6-Dibrompyridin, 8,19 g (80,9 mmol) Triethylamin, 0,470 g (1,79 mmol) Triphenylphosphin, 0,184 g (0,82 mmol) Palladiumdiazetat und 9,36 g (40,4 mmol) Silberoxid werden in 120 ml abs. DMF 2 Stunden bei 105 °C gerührt.
Die abgekühlte Lösung wird über Hyflo filtriert, mit EE nachgewaschen und das Lösungsmittel abgezogen. Der Rückstand wird aus Acetonitril/Akivkohle umkristallisiert.
Ausbeute: 4,37 g hellbeige Kristalle (35% der Theorie)
Schmelzpunkt: 154 - 159 °C (Acetonitril)
Dünnschichtchromatogramm:
CHCl₃:Ac 7:3 R_{F} = 0,95
PE:EE 40:1 R_{F} = 0,30
Bz:Et₂O 1:1 R_{F} = 0,90
¹H-NMR: (Benzol-d6)
δ(ppm): 8,05 (d, 2H, Bz-H3); 7,50 (d, 2H, Ph-H2); 7,40 (d, 2H, Bz-H2*); 7,10-7,30 (m, 3H, Py-H3 + Ph-H3); 7,05 (d, 1H, Ph-H4); 6,90 (d, 1H, Py-H5*); 6,70 (m, 1H, Py-H4*)
¹³C-NMR: (Trifluoressigsäure-d6)
δ(ppm): 159,29 (s, Py-C2); 149,96 (s, Py-C6); 148,17 (d, Py-C4*); 141,49 (s, Ph-C1);
137,13 (s, Bz-C1); 132,56 (d, Py-C3*); 131,96 (d, Bz-C3 + Bz-C5); 131,85 (d, Ph-C3 + Ph-C5); 131,562 (s, Bz-C4*); 131,46 (s, Ph-C4*); 130,57 (d, Bz-C2 + Bz-C6); 129,73 (d, Ph-C2 + Ph-C6); 127,39 (d, Py-C5*)

### 6-(4-Biphenyl)-2-pyridinamin

2,65 g (8,54 mmol) 4-Biphenyl-2-brompyridin und 1,82 g (46,7 mmol) Natriumamid in 200 ml Ammoniak werden in einem Autoklaven 60 Minuten bei 0 °C gerührt. Überschüssiges Amid wird mit 2,49 g (46,6 mmol) Ammonchlorid beseitigt und der Ammoniak langsam verdampft. Der Rückstand wird zwischen Wasser und EE verteilt, 7x mit insgesamt 700 ml EE extrahiert, die organische Phase 2x mit Wasser gewaschen, über Natriumsulfat getrocknet und das Lösungsmittel abgezogen.
Das Rohprodukt wird 2x mit jeweils 20 ml HCl-gesättigtem Methanol digeriert und eingedampft. Der Rückstand wird mit 40 ml Benzol heiß digeriert, abfiltriert und mit heißem Benzol gewaschen. Das reine Amin wird durch Verteilung zwischen Methylenchlorid und gesättigter Natriumhydrogencarbonatlösung freigesetzt, die organische Phase 2x mit Wasser gewaschen, getrocknet und das Lösungsmittel abgezogen.
Ausbeute: 1,07 g beige Kristalle (51% der Theorie)
Schmelzpunkt: 240 - 243 °C (Benzol)
Dünnschichtchromatogramm: Bz:Et₂O 1:1 R_{F} = 0,50
¹H-NMR: (CDCl₃)
δ(ppm): 8,05 (d, 2H, Bz-H3); 7,70 (d, 2H, Bz-H2); 7,65 (dd, 2H, Ph-H2); 7,35 - 7,55 (m, 4H, Ph-H4 + Ph-H3 + Py-H4); 7,15 (d, 1H, Py-H3); 6,45 (d, 1H, Py-H5)
¹³C-NMR: (CDCl₃)
δ(ppm): 158,27 (s, Py-C6); 155,57 (s, Py-C2); 141,20 (s, Ph-C1); 140,65 (s, Bz-C1);
138,52 (s, Bz-C4); 138,31 (d, Py-C4); 128,72 (d, Bz-C3 + Bz-C5); 127,33 (s, Ph-C4); 127,17 (d, Ph-C2 + Ph-C6); 127,15 (d, Ph-C3 + Ph-C5);
126,99 (d, Bz-C2 + Bz-C6); 110,77 (d, Py-C3); 107,11 (d, Py-C5)

### Beispiel 3

### 6-Chlor-N-{6-[3-(4-fluorphenoxy)phenyl]-2-pyridinyl}-4-hydroxy-2-methyl-2H-thieno[2,3-e]-1,2-thiazin-3-carboxamid-1,1-dioxid

Ein Gemisch aus 1.00 g (3.57 mMol) 6-(3-(4-Fluorphenoxy)phenyl)-2-pyridinamin und 1.11 g (3.57 mMol) 6-Chlor-4-hydroxy-2-methyl-2H-thieno[2,3-e]-1,2-thiazin-3-carbonsäuremethylester-1,1-dioxid in 29 ml absolutem Xylol wird 22 Stunden zum Sieden erhitzt.
Das unter Kühlung ausgefallene Rohprodukt wird abfiltriert und aus Acetonitril/Aktivkohle umkristallisiert.
Ausbeute: 1.19 g gelbe Kristalle (60% d.Th.)
DC: LM...CH₂Cl₂:MeOH = 40:1; 0.4
Fp.: 166-169°C (Acetonitril)
¹H-NMR: (DMSO-d₆)
d(ppm): 8.14-7.70 (m, 6H, ThH, BzH2,6, PyH3,4,5); 7.52 (dd, 1H, BzH5); 7.30-7.00 (m, 5H, BzH4, FBzH2,3,5,6); 2.95 (s, 3H, N-CH₃)
¹³C-NMR: (DMSO-d₆)
d(ppm): 166.3, 158.2 (d), 157.5, 155.5, 152.8, 152.6, 152.5, 150.1, 140.1, 139.1, 137.4, 136.3, 134.9, 130.4, 123.1, 121.7, 120.4(d), 119.1, 116.7, 116.5(d), 115.0, 110.3, 39.4
Das Ausgangsmatrerial kann wie folgt hergestellt werden:

### Tributyl-[3-(4-fluorphenoxy)phenyl]stannan

Zu 14.23 g (53.3 mMol) 1-Brom-3-(4-fluorphenoxy)benzol in 150 ml absolutem Tetrahydrofuran werden bei -70°C 21.3 ml (53.3 mMol) einer 2.5 m Lösung von n-Butyllithium in n-Hexan getropft, eine Stunde gerührt und mit 17.35 g (53.3 mMol) Tributylzinnchlorid bei -70°C versetzt.
Nach 30 Minuten wird auf Raumtemperatur erwärmt, das Lösungsmittel abgezogen, zwischen 250 ml Wasser und 200 ml Diethylether verteilt, und noch einmal mit 200 ml Diethylether extrahiert. Die organische Phase wird über Na₂SO₄/Aktivkohle getrocknet, filtriert und das Lösungsmittel abgezogen.
Ausbeute: 25.54 g gelbliches Öl (90% d.Th.)
DC: LM...CCl₄; 0.65
¹H-NMR: (CDCl₃)
d(ppm): 7.29 (ddd, 1H, BzH5); 7.19 (ddd, 1H, BzH6); 7.13-6.95 (m, 5H, BzH2, FBzH2,3,5,6); 6.87 (ddd, 1H, BzH4); 1.60-1.40 (m, 6H, BuH2); 1.40-1.25 (m, 6H, BuH3); 1.11-0.96 (m, 6H, BuH1); 0.87 (t, 9H, BuH4)
¹³C-NMR: (CDCl₃)
d(ppm): 158.4 (d), 156.9, 153.1 (d), 144.1, 131.1 (t), 129.0 (t), 126.0 (t), 120.1 (d), 117.8, 116.0 (d), 29.0 (t), 27.2 (t), 13.5, 9.5 (t)

### 2-Brom-6-(3-(4-fluorphenoxy)-phenyl]-pyridin

10.94 g (44.3 mMol) 2,6-Dibrompyridin, 149 mg (664 *µ*Mol) Palladium-(II)-acetat und 349 mg (1.33 mMol) Triphenylphosphin in 235 ml abs. DMF wird unter Stickstoffatmosphäre bei 80°C mit 23.67 g (44.3 mMol) Tributyl-[3-(4-fluorphenoxy)phenyl]stannan in 12 ml abs. DMF versetzt und 10 Stunden gerührt.
Nach abziehen des Lösungsmittels wird der Rückstand zwischen 400 ml Wasser und 200 ml Diethylether verteilt und die wäßrige Phase noch zweimal mit je 200 ml Diethylether extrahiert. Die vereinten organischen Phasen werden dreimal mit je 100 ml Wasser gewaschen, über Na₂SO₄/Aktivkohle getrocknet, filtriert und das Lösungsmittel abgezogen.
Das Rohprodukt wird einer chromatographischen Trennung (400 g KG60, PE:Bz=3:2) unterworfen.
Ausbeute: 9.00 g farblose Kristalle 59% d.Th.
DC: LM...CCl₄:Et₂O = 10:1; 0.65
Fp.: 80-81°C (SC)
¹H-NMR: (CDCl₃)
d(ppm): 7.72 (ddd, 1H, BzH6), 7.66-7.60 (m, 2H, PyH5, BzH2), 7.57 (dd, 1H, PyH4), 7.41 (dd, 1H, BzH5), 7.41 (dd, 1H, PyH3), 7.10-6.97 (m, 5H, BzH4, FBzH2,3,5,6)
¹³C-NMR: (CDCl₃)
d(ppm): 158.7 (d), 158.0, 157.6, 152.7 (d), 142.0, 139.5, 138.9, 130.0, 126.6, 121.7, 120.3 (d), 119.1 (d), 116.9, 116.5, 116.0

### 6-[3-(4-Fluorphenoxy)-phenyl]-2-pyridinamin

7.00 g (20.3 mMol) 2-Brom-6-[3-(4-fluorphenoxy)-phenyl]-pyridin und 4.76 g (122.1 mMol) Natriumamid in 400 ml flüssigem Ammoniak werden 30 Minuten bei 0°C gerührt und mit 6.52 g (121 mMol) Ammoniumchlorid versetzt.
Der Ammoniak wird abgedampft und der Rückstand zwischen 250 ml Wasser und 100 ml Diethylether verteilt, die wäßrige Phase ein weiteres Mal mit 100 ml Diethylether extrahiert, die vereinten organischen Phasen über Na₂SO₄/Aktivkohle getrocknet, filtriert und das Lösungsmittel abgezogen.
Das Rohprodukt wird einer Flashchromatographie (134 g KG60, Bz:Et₂O=3:1) unterworfen.
Ausbeute: 1.63 g braunes Öl (29% d.Th.)
DC: LM...Bz:Et₂O = 1:1; 0.55
¹H-NMR: (CDCl₃)
d(ppm): 7.67 (ddd, 1H, BzH6); 7.59 (dd, 1H, BzH2); 7.46 (dd, 1H, BzH5); 7.38 (dd, 1H, PyH4); 7.08-6.93 (m, 6H, BzH4, PyH5, FBzH2,3,5,6); 6.44 (d, 1H, PyH3)
¹³C-NMR: (CDCl₃)
d(ppm): 158.6 (d), 158.2, 157.8, 155.0, 152.9, 141.6, 138.3, 129.8, 121.6, 120.3 (d), 118.4, 116.8, 116.2 (d), 110.8, 107.4

### Beispiel 4

### 6-Chlor-N-{6-[5-(4-fluorphenoxy)-2-furyl]-2-pyridinyl}-4-hydroxy-2-methyl-2H-thieno[2,3-e]-1,2-thiazin-3-carboxamid-1,1-dioxid

114 mg (422 µmol) 6-5-(4-Fluorphenoxy)-2-furyl-2-pyridinamin und 131 mg (422 µmol) 6-Chlor-4-hydroxy-2-methyl-2H-thieno2,3-e-1,2-thiazin-3-carbonsäure-methylester-1,1-dioxid werden in 3,4 ml Xylol 20 Stunden zum Sieden erhitzt. Das Lösungsmittel wird abgezogen und der Rückstand aus Acetonitril/Aktivkohle umkristallisiert.
Ausbeute: 191 mg gelbe Kirstalle (83% d.Th.)
DC: LM ... CH₂Cl₂:MeOH = 40:1; 0,65
Fp.: 186-195°C
¹H-NMR: (Aceton-d6)
d(ppm): 8.05-7.83 (m, 2H, PyH3,4); 7.57 (s, 1H, ThH); 7.45 (dd, 1H, PyH5); 7.33-7.15 (m, 4H, BzH2,3,5,6); 7.07 (d, 1H, FuH3); 5.80 (d, 1H, FuH4); 3.10 (s, 3H, CH3)
¹³C-NMR: (Aceton-d6)
d(ppm): 169.1; 169.0; 161.3 (d); 159.8; 157.0; 154.3; 152.2 (d); 149.0; 147.0; 141.4; 140.3; 139.4; 125.0; 121.3 (d); 118.5 (d); 116.1; 114.8; 113.2; 112.8; 92.9; 41.4
Das Ausgangsmaterial kann wie folgt hergestellt werden:

### 2-Brom-6-(2-furyl)pyridin

30,0 g (268,12 mMol) 2-Furanborsäure, 42,35 g (178,75 mMol) 2,6-Dibrompyridin, 54,26 g (536,24 mMol) Triethylamin, 2,8 g (10,73 mMol) Triphenylphosphin und 1,2 g (5,36 mMol) Palladiumdiacetat werden in 800 ml N,N-Dimethylformamid suspendiert und drei Stunden unter Stickstoffatmosphäre bei 100°C gerührt.
Nach Abziehen des Lösungsmittels wird der Rückstand zwischen 400 ml Dichlormethan und 200 ml 10% igem wäßrigen Ammoniak verteilt. Die wäßrige Phase wird einmal mit 200 ml Dichlormethan rückgeschüttelt, die gesammelten organischen Phasen mit 200 ml Wasser gewaschen, mit Natriumsulfat getrocknet, filtriert und das Lösungsmittel abgezogen.
Zur Reinigung wird das Rohproduk: über 1000 g Kieselgel KG 60 mit Benzol chromatographiert und im Feinvakuum destilliert.
Ausbeute: 31,0 g farblose Flüssigkeit (78% d.Th.)
DC: PE:EE =2:1 ; 0,35
Kp: 80-85°C/0,1 mbar
n_{D}²⁰: 1,6468
¹H-NMR: (CDCl₃)
d(ppm): 7,54(m, 3H, Py-H3, Py-H4, Fu-H5); 7,28(d, 1H, Py-H5, ³J_{H,H}=8Hz); 7,10(d, 1H, Fu-H3, ³J_{H,H}=4Hz); 6.51 (dd, 1H, Fu-H4, ³J_{H,H3}=4Hz, ³J_{H,H5}=2Hz)
¹³C-NMR:(CDCl₃)
d(ppm): 151,9(s, Py-C6); 149,9(s, Fu-C2); 143,6(d, Fu-C5); 141,7(s, Py-C2); 138,6(d, Py-C4); 125,7(d, Py-C3); 116,8(d, Py-C5); 112,0(d, Fu-C4); 109,9(d, Fu-C3)

### 6-(2-Furyl)-2-pyridinamin

20,0 g (89,26 mMol) 2-Brom-6-(2-furyl)-pyridin und 13,8 g (357,05 mMol) Natriumamid werden in 1700ml Ammoniak gelöst und 15 Minuten bei -33°C gerührt, mit 18,8 g (357,05 mMol) Ammonchlorid gequencht und der Ammoniak verdampfen lassen. Der Rückstand wird zwischen 400 ml Diethylether und 400 ml Wasser verteilt und die wässrige Phase noch einmal mit 200 ml Diethylether extrahiert. Die gesammelten organischen Phasen werden mit Natriumsulfat getrocknet, filtriert und das Lösungsmittel abgezogen.
Das Rohprodukt wird säulenchromatographisch über 400 g Kieselgel KG 60 mit CH₂Cl₂:Et₂O = 2:1 gereinigt.
Ausbeute: 8,3 g blaßgelbe Kristalle (58% d.Th.)
DC: CH₂Cl₂:Et₂O =2:1 ; 0,35
Fp: 66-68°C (chromatographiert)
¹H-NMR: (CDCl₃)
δ(ppm): 7,49(d, 1H, Fu-H5, ³J_{H,H}=2Hz); 7,45(dd, 1H, Py-H4, ³J_{H,H3}=³J_{H,H5}=8Hz); 7,07(d, 1H, Py-H5, ³J_{H,H}=8Hz); 6,92(d, 1H, Fu-H3, ³J_{H,H}=4Hz); 6,49(dd, 1H, Fu-H4, ³J_{H,H3}=4Hz, ³J_{H,H5}=2Hz); 6,37(d, 1H, Py-H3, ³J_{H,H}=8Hz); 4,56(s, 2H, Py-NH₂)
¹³C-NMR:(CDCl₃)
δ(ppm): 158,1 (s, Py-C2); 153,6(s, Py-C6); 147,5(s, Fu-C2); 142,8(d, Fu-C5); 138,2(d, Py-C4); 111,7(d, Fu-C4); 108,9(d, Py-C5); 107,9(d, Fu-C3); 107,2(d, Py-C3)

### N-(1,1-Dimethylethoxycarbonyl)-N-[6-(2-Furyl)-2-pyridinyl]-carbaminsäure-1,1-dimethylethylester

6,0 g (37,46 mMol) 6-(2-Furyl)-2-pyridinamin werden mit 16,35 g (74,92 mMol) Dikohlensäure-bis(1,1-dimethylethyl)-ester und 10,0 g (74,92 mMol) Kaliumcarbonat in 200 ml abs. Dioxan mit einer katalytischen Menge N,N-Dimethyl-4-pyridinamin 90 Minuten zum Sieden erhitzt.
Nach Erkalten der Lösung wird das Kaliumcarbonat abfiltriert und das Lösungsmittel im Vakuum abgezogen. Der Rückstand wird in 100 ml 2n wäßriger Salzsäure aufgenommen und dreimal mit 100 ml Diethylether extrahiert. Die gesammelten organischen Phasen werden einmal mit 100 ml 2n wäßriger Natronlauge und zweimal mit Wasser gewaschen, über Natriumsulfat getrocknet, filtriert und das Lösungsmittel im Vakuum abgezogen.
Der Rückstand wird durch Digerieren mit Diisopropylether gereinigt.
Ausbeute: 11,5 g beige Kristalle (85% d.Th.)
DC: PE:EE = 4:1; 0,5
Fp: 99-101°C (Diisopropylether)
¹H-NMR: (CDCl₃)
δ(ppm):7,73(dd, 1H,Py-H4, ³J_{H,H3}=³J_{H,H5}=8Hz); 7,57(d, 1H, Py-H3, ³J_{H,H}=8Hz); 7,49(d, 1H, Fu-H5, ³J_{H,H}=2Hz); 7,11 (d, 1H, Py-H5, ³J_{H,H}=8Hz); 7,00(d, 1H, Fu-H3, ³J_{H,H}=4Hz); 6,50(dd, 1H, Fu-H4, ³J_{H,H3}=4Hz, ³J_{H,H5}=2Hz); 1,45(s, 18H, -CH₃)
¹³C-NMR:(CDCl₃)
δ(ppm): 152,9(s, Py-C2); 151,9(s, C=O); 151,1(s, Py-C6); 148,2(s, Fu-C2); 143,1(d, Fu-C5); 138,1(d, Py-C4); 118,9(d, Py-C3); 116,1(d, Fu-C3); 111,9(d, Fu-C4); 108,9(d, Py-C5); 82,7(s, C-CH₃); 27,7(q, C-CH₃)

### N-[6-(2-Furyl)-2-pyridinyl]-carbaminsäure-1,1-dimethylethylester

11,5 g (31,91 mMol) N-(1,1-Dimethylethoxycarbonyl)-N-[6-(2-furyl)-2-pyridinyl]-carbaminsäure-1,1-dimethylethylester werden in 100 ml abs. Methanol mit 5,75 ml (31,91 mMol) Natriummethanolat 30% ig in abs. Methanol versetzt und 60 Minuten bei Raumtemperatur gerührt. Dann werden noch 2,9 ml (16,09 mMol) Natriummethanolat 30% ig in abs. Methanol zugegeben, weitere 30 Minuten gerührt und das Lösungsmittel im Vakuum abgezogen.
Der Rückstand wird mit 100 ml 2n wäßriger Salzsäure aufgenommen und dreimal mit 100 ml Diethylether extrahiert. Die gesammelten organischen Phasen werden zweimal mit 100 ml Wasser gewaschen, über Natriumsulfat getrocknet und das Lösungsmittel im Vakuum abgezogen.
Der Rückstand wird säulenchromatographisch über 400 g KG 60 mit Benzol gereinigt.
Ausbeute: 7,3 g farbloser Schaum (88% d.Th.)
DC: PE:EE = 4:1; 0,6
¹H-NMR: (CDCl₃)
δ(ppm): 7,81 (d, 1H, Py-H3, ³J_{H,H}=8Hz); 7,66(dd, 1H, Py-H4, ³J_{H,H3}=³J_{H,H5}=8Hz); 7,48(d, 1H, Fu-H5, ³J_{H,H}=2Hz); 7,32(d, 1H, Py-H5, ³J_{H,H}=8HZ); 7,30(s, 1H, Py-NH); 6,93(d, 1H, Fu-H3, ³J_{H,H}=4Hz); 6,50(dd, 1H, Fu-H4, ³J_{H,H3}=4Hz, ³J_{H,H5}=2Hz); 1,49(s, 9H, -CH₃)
¹³C-NMR:(CDCl₃)
δ(ppm): 153,2(s, Py-C2); 152,4(s, C=O); 151,6(s, Py-C6); 147,5(s, Fu-C2); 143,1(d, Fu-C5); 138,7(d, Py-C4); 113,2(d, Py-C3); 111,9(d, Fu-C3); 110,5(d, Fu-C4); 108,5(d, Py-C5); 80,8(s, C-CH₃); 28,1(q, C-CH₃)

### N-[6-(5-Brom-2-furyl)-2-pyridinyl]-carbaminsäure-1,1-dimethylethylester

7,0 g (26,89 mMol) N-[6-(2-Furyl)-2-pyridinyl]-carbaminsäure-1,1-dimethylethylester werden mit 4,8 g (26,89 mMol) N-Bromsuccinimid in 50 ml abs. Tetrachlormethan zwei Stunden zum Sieden erhitzt. Nach Erkalten wird das Reaktionsgemisch filtriert und das Filtrat vom Lösungsmittel befreit.
Der Rückstand wird aus Methanol/Aktivkohle umkristallisiert.
Ausbeute: 7,5 g farblose Kristalle (82% d.Th.)
DC: PE:EE = 4:1 ; 0,6
Fp: 115-117°C (MeOH)
¹H-NMR: (CDCl₃)
δ(ppm): 7,81 (d, 1H, Py-H3, ³J_{H,H}=8Hz); 7,67(dd, 1H, Py-H4, ³J_{H,H3}=³J_{H,H5}=8Hz); 7,48(s, 1H, Py-NH); 7,30(d, 1H, Py-H5, ³J_{H,H}=8Hz); 6,88(dd, 1H, Fu-H4, ³J_{H,H3}=4Hz, ³J_{H,H5}=2Hz); 6,39(d, 1H, Fu-H3, ³J_{H,H}=4Hz); 1,49(s, 9H, -CH₃)
¹³C-NMR:(CDCl₃)
δ(ppm): 154,9(s, Py-C2); 152,2(s, C=O); 151,7(s, Py-C6); 146,3(s, Fu-C5); 138,7(d, Py-C4); 122,7(s, Fu-C2); 113,6(d, Py-C3); 112,8(d, Fu-C4); 110,8*(d, Py-C5); 110,7*(d, Fu-C3); 80,7(s, C-CH₃); 28,0(q, C-CH₃)

### 6-(5-Brom-2-furyl)-2-(dimethyl-ethoxycarbonyl-amino)-pyridin-1-oxid

7,5 g (22,11 mMol) N-[6-(5-Brom-2-furyl)-2-pyridinyl]-carbaminsäure-1,1-dimethyl-ethylester werden mit 9,02 g (28,75 mMol) 3-Chlorperbenzoesäure 55% ig in 150 ml abs. Trichlormethan 30 Minuten bei Raumtemperatur gerührt.
Die Lösung wird zweimal mit je 100 ml einer gesättigten wäßrigen Natrium-hydrogencarbonatlösung und zweimal mit je 50 ml Wasser gewaschen. Die organische Phase wird mit Natriumsulfat getrocknet, filtriert und das Lösungsmitttel im Vakuum abgezogen.
Der Rückstand wird aus Aceton/Aktivkohle umkristallisiert.
Ausbeute: 3,7 g farblose Kristalle (48% d.Th.)
DC: PE:EE = 4:1; 0,55
Fp: 120-122°C (Aceton)
¹H-NMR:(CDCl₃)
δ(ppm): 9,45( s, 1H, Py-NH); 8,00(d, 1H, Py-H3, ³J_{H,H}=8Hz); 7,91 (d, 1H, Fu-H4, ³J_{H,H3}=4Hz,); 7,50(d, 1H, Py-H5, ³J_{H,H}=8Hz); 7,31 (dd, 1H, Py-H4, ³J_{H,H3}=³J_{H,H5}=8Hz); 6,52(d, 1H, Fu-H3, ³J_{H,H}=4Hz); 1,53(s, 9H, - CH₃)
¹³C-NMR:(CDCl₃)
δ(ppm): 151,4(s, C=O); 147,2(s, Py-C2); 144,7(s, Py-C6); 137,0(s, Fu-C5); 126,5(d, Py-C4); 124,5(s, Fu-C2); 118,6(d, Fu-C4); 114,2(d, Fu-C3); 113,5(d, Py-C3); 109,6(d, Py-C5); 81,9(s, C-CH₃); 28,0(q, C-CH₃)

### 6-[5-(4-Fluorphenoxy)-2-furyl]-2-pyridinamin, 1-oxid

2.37 g (6.66 mMol) 6-(5-Brom-2-furyl)-2-(dimethyl-ethoxycarbonyl-amino)-pyridin-1-oxid und 2.95 g (22.0 mMol) Natrium-4-fluorphenolat werden in 23 ml absolutem Dimethylsulfoxid 13 Stunden bei 100°C gerührt, wobei nach 8 bzw. 11 Stunden noch je 0.90 g (6.66 mMol) Natrium-4-fluorphenolat zugegeben werden.
Das Reaktionsgemisch wird mit 500 ml Wasser verdünnt, das Produkt mit 4x200 ml Diethylether extrahiert und die vereinten organischen Phasen mit 2x100 ml 2n Natriumhydroxidlösung sowie 100 ml Wasser gewaschen. Das Extrakt wird über Na₂SO₄/Aktivkohle getrocknet, filtriert und das neue Lösungsmittel abgezogen. Das Rohprodukt wird aus Acetonitril/Aktivkohle umkristallisiert.
Ausbeute: 0.42 g hellbeige Kristalle (22% d.Th.)
DC: LM...Bz:Et₂O=1:1; 0.2
Fp.: 195-201°C (Acetonitril, Zers.)
¹H-NMR: (CDCl3)
δ(ppm): 7.67 (dd, 1H, PyH4); 7.58 (d, 1H, FuH3); 7.24-7.02 (m, 6H, PyH3,5, BzH2,3,5`6); 5.63 (d, 1H, FuH4)
¹³C-NMR: (CDCl3)
δ(ppm): 161.0; 159.9 (d); 156.2; 150.3 (d); 137.2; 132.8; 130.5; 120.6; 120.1; 116.7; 109.7; 107.0; 90.0

### 6[5-(4-Fluorphenoxy)-2-furyl]-2-pyridinamin

226 mg (789 *µ*Mol) 6-[5(4-Fluorphenoxy)-2-furyl]-2-pyridinamin-1-oxid werden mit 226 mg (4.05 mMol) Eisenpulver in 4.5 ml Eisessig p.a. unter Stickstoffatmosphäre bei 80°C zwei Stunden heftig gerührt, wobei nach einer Stunde noch 100 mg (1.80 mMol) Eisenpulver zugegeben werden.
Nach dem Abziehen des Lösungsmittels wird der Rückstand zwischen 20 ml Diethylether und 20 ml verdünnter Salzsäure verteilt. Man extrahiert die wäßrige Phase noch mit 3x15 ml Diethylether, wäscht die vereinten organischen Phasen mit 2x20 ml 1N Salzsäure sowie mit 20 ml gesättigter Natriumhydrogencarbonatlösung. Die organische Phase wird über Na₂SO₄ getrocknet, filtriert und das Lösungsmittel abgezogen und das Rohprodukt einer Flashchromatographie über 11 g Kieselgel KG 60 mit Benzol:Ether = 1:1 unterworfen.
Ausbeute: 178 mg farblose Kristalle (83% d.Th.)
DC:
LM...Et₂O:MeOH=50:1; 0.6
LM...Bz:Et₂O=1:1; 0.3
Fp.: 98-100°C (SC aus Benzol/Diethylether)
¹H-NMR: (CDCl3)
δ(ppm): 7.41 (dd, 1H, PyH4); 7.15-6.96 (m, 4H, BzH2,3,5,6); 6.94 (dd, 1H, PyH5);6,90 (d,1H,FuH3); 6,33 (dd,1H,PyH3); 5,60(d,1H,FuH4); 4,59 (sbreit, 2H,NH2)
13C-NMR: (CDCl3)
δ(ppm: 159.2(d); 158,2, 157.0; 152.3 (d); 147.3; 146.1; 138.2; 118,8 (d); 116.3 (d); 109.4; 108.2; 106.9; 90.8

### Beispiel 5:

### N-[6-(2-Benzo[b]furyl)-2-pyridinyl]-6,7-dichlor-4-hydroxy-2-methyl-2H-thieno-[2,3-e][1,2]thiazin-3-carboxamid-1,1-dioxid

428 mg (2,03 mmol) 6-(2-Benzo[b]furyl)-2-pyridinamin und 700 mg (3,99 mmol) 6,7- Dichlor-4-hydroxy-2-methyl-2H-thieno-[2,3-e][1,2]thiazin-3-carbonsäure-methylester-1,1-dioxid werden in 7ml abs. Xylol 9 Stunden zum Sieden erhitzt. Die Lösung wird abgekühlt, mit 25 ml Diethylether verdünnt, der Niederschlag abfiltriert und mehrmals mit kaltem Diethylether digeriert. Dieses Rohprodukt wird aus Chloroform/Aktivkohle umkristallisiert.
Ausbeute: 734 mg gelbe Kristalle (57% der Th.)
Fp.: 225-227°C
Dünnschichtchromatogramm: PE:EtOH=2:1 R_{F}= 0,25
¹H-NMR: (DMSO)
δ(ppm): 8,09-7,88 (m, 2H, Py-H5,Bzfu-H7); 7,74-7,53 (m, 4H, Bzfu-H3,4,Py-H3,4); 7,43-7,21 (m, 2H, Bzfu-H5,6); 3,02 (s, 3H, CH3)

### Beispiel A:

Die Bildung von Prostaglandin D2 durch Neutrophile wurde als Maß für die Cyclooxygenase-Aktivität verwendet und die Bildung von Leukotrien B4 als Maß für die 5-Lipoxygenase-Aktivität.
Männlichen Sprague Dawley Ratten (250-300 g) wurde lamda - Carrageenan 1 mg intraperitoneal (gelöst in 0.5 ml dest. Wasser) injiziert.
Nach 16 Stunden wurden die Ratten durch Exposition in Diethylether getötet. 15 ml eiskalte Hanks balanced salt solution (HBSS) wurden i.p. injiziert, die Neutrophilen durch Absaugen geerntet (10 ml), zentrifugiert (5 min, 100g, 4°C), die überstehende Lösung dekantiert und die Zellen in HBSS resuspendiert bei 4 °C bis zu einer Konzentration von 5x10⁶ Zellen/ml.
400 *µ*l Zellsuspension (2x10⁶ Zellen), 0,5*µ*l Verbindung gelöst in DMSO und 49,5 *µ*l HBSS wurden 5 min bei 37°C inkubiert. Dann wurden 50 *µ*l A23187 (2*µ*mol/l Endkonzentration) zugefügt und anschließend wiederum 5 min bei 37°C inkubiert.
Die Reaktion wurde durch Zentrifugieren 10000g, 3s gestoppt und die überstehende Flüssigkeit in vorgekühlte Plastikröhrchen übergeführt und vor dem Beginn der Radioimmunoassay-Messung im Eisbad max. 1 h belassen.
PGD2 und LTB4 wurde nach Verdünnung mit HBSS mit Hilfe kommerzieller RIA-Kits gemessen.
Als Vergleichssubstanz diente 6-Chlor-4-hydroxy-2-methyl-N-(2-pyridyl)-2H-thieno(2,3-e)1,2-thiazin-3carboxamid-1,1-dioxid ("Lornoxicam", Vbg.A)

| Vbg | IC50 (µmol/l) | |
|---|---|---|
| | PGD2 | LTB4 |
| A | 0.02 | >10 |
| 1 | 0.049 | 2 |
| 2 | 3.5 | 1.4 |
| 3 | 0.45 | 1.7 |
| 4 | 1.7 | 1.3 |
| 5 | 0.036 | 1.3 |

## Patentansprüche

1. Thienothiazinderivate der Formel I 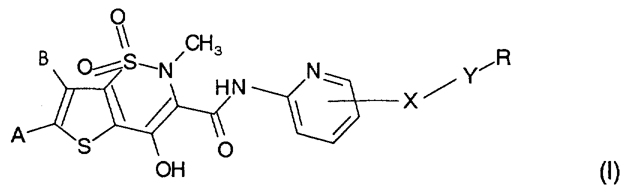 in der X eine Einfachbindung oder einen 5 - 12 gliedrigen, mono- oder polycyclischen, gegebenenfalls teilweise hydrierten Aryl- oder Heteroarylrest, der gegebenfalls durch Halogen, Niederalkyl oder Niederalkoxy substituiert sein kann,
Y eine Einfachbindung oder ein Heteroatom
R einen mono- oder polycyclischen, gegebenenfalls teilweise hydrierten 5 - 12 gliedrigen Aryl- oder Heteroarylrest bedeutet, der gegebenenfalls ein- oder mehrfach durch Halogen, Niederalkyl oder Niederalkoxy substituiert sein kann, A Halogen
und
B Wasserstoff oder Halogen bedeutet.

2. Thienothiazinderiviate der Formel (I) nach Anspruch 1, dadurch gekennzeichnet, daß der Substituent in 6 - Stellung mit dem Pyridin verknüpft ist.

3. Thienothiazinderivate der Formel (I) nach Anspruch 1, dadurch gekennzeichnet, daß X eine Einfachbindung oder einen Phenyl- oder Furylrest bedeutet.

4. N-[6-(2-Benzo[b]furyl)-2-pyridinyl]-6,7-dichlor-4-hydroxy-2-methyl-2H-thieno-[2,3-e][1,2]thiazin-3-carboxamid-1,1-dioxid

5. Verfahren zur Herstellung von Verbindungen der Formel (I) 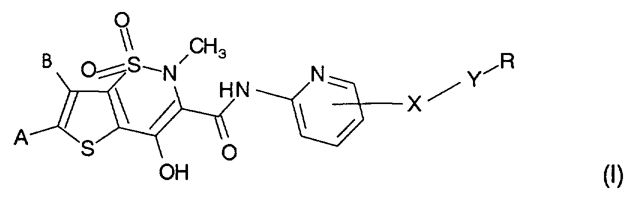 in der X eine Einfachbindung oder einen 5 - 12 gliedrigen, mono- oder polycyclischen, gegebenenfalls teilweise hydrierten Aryl- oder Heteroarylrest, der gegebenfalls durch Halogen, Niederalkyl oder Niederalkoxy substituiert sein kann,
Y eine Einfachbindung oder ein Heteroatom
R einen mono- oder polycyclischen, gegebenenfalls teilweise hydrierten 5 - 12 gliedrigen Aryl- oder Heteroarylrest bedeutet, der gegebenenfalls ein- oder mehrfach durch Halogen, Niederalkyl oder Niederalkoxy substituiert sein kann, A Halogen
und
B Wasserstoff oder Halogen bedeutet, dadurch gekennzeichnet, daß man eine Verbindung der Formel (II) 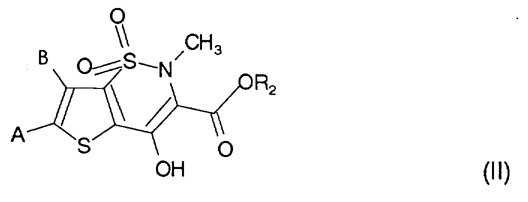 in der R1 eine geradkettige oder verzweigte Alkylgruppe mit 1 - 4 C-Atomen bedeutet und A und B die oben genannte Bedeutung haben, mit einer Verbindung der Formel (III) 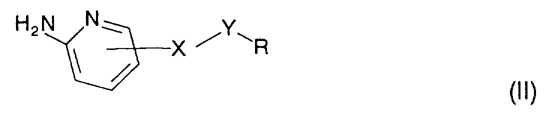 in der X, Y, und R die oben genannte Bedeutung haben, umsetzt und die so erhaltenen Verbindungen der Formel (I) gegebenenfalls in ihre pharmazeutisch verwendbaren Salze überführt.

6. Verfahren nach Anspruch 5, dadurch gekennzeichnet, daß die Umsetzung in Gegenwart einer starken Base durchgeführt wird.

7. Pharmazeutische Präparate enthaltend Verbindungen der allgemeinen Formel (I) nach Anspruch 1, sowie deren Salze in Kombination mit üblichen galenischen Hilfs- und/oder Tragerstoffen.

8. Pharmazeutische Präparate nach Anspruch 7 in Kombination mit anderen therapeutisch wertvollen Verbindungen sowie Hilfs- und/oder Trägerstoffen.

9. Verbindungen nach Anspruch 1 zur Verwendung als Wirkstoffe für Arzneimittel zur Behandlung von Entzündungen und Schmerzzustanden.

10. Verbindungen nach Anspruch 1 als Antiinflammatorikum und Analgeticum.
